# EUROPEAN PATENT SPECIFICATION

(11) **EP 2 479 318 B1**
(45) Date of publication and mention of the grant of the patent: **16.10.2013**
(21) Application number: 12164428.0
(22) Date of filing: 27.07.2009
(51) Int. Cl.: C25D 11/26, A61L 27/04, A61L 27/06, A61L 27/32, A61F 2/00

(54) **Metal substrates modified with silicon-based biomimetic treatment for the osteointegration thereof.**
Mit biomimetischer Behandlung auf Siliciumbasis modifizierte Metallsubstrate zu deren Osteointegration
Substrats métalliques modifiés par un traitement biomimétique à base de silicium pour l'ostéointégration correspondante

(30) Priority: 29.07.2008 IT MI20081399
(43) Date of publication of application: 25.07.2012
(62) Divisional of application: 09802579.4
(73) Proprietor: Politecnico Di Milano, 20133 Milano (IT)
(72) Inventor: Chiesa, Roberto, 22029 Uggiate Trevano (IT); Cigada, Alberto, 20123 Milano (IT); Della Valle, Cinzia, 23030 Chiuro (IT); Rondelli, Gianni, 40127 Bologna (IT); Candiani, Gabriele, 20123 Milano (IT); Giordano, Carmen, 80124 Napoli (IT)
(74) Representative: Asensio, Raffaella Consuelo

(56) References cited:
- EP-B1- 1 515 759
- US-A- 5 478 237
- LIU X ET AL: "Surface modification of titanium, titanium alloys, and related materials for biomedical applications", MATERIALS SCIENCE AND ENGINEERING R: REPORTS, ELSEVIER SEQUOIA S.A., LAUSANNE, CH, vol. 47, no. 3-4, 24 December 2004 (2004-12-24), pages 49-121, XP004722113, ISSN: 0927-796X
- PARK ET AL: "Surface characteristics of titanium anodized in the four different types of electrolyte", ELECTROCHIMICA ACTA, ELSEVIER SCIENCE PUBLISHERS, BARKING, GB, vol. 53, no. 2, 11 October 2007 (2007-10-11), pages 863-870, XP022346644, ISSN: 0013-4686

## Description

### Technical field

The present invention relates to surface modification of titanium, tantalum and respective alloys by means of an electrochemical treatment of anode oxidation. This method is particularly useful for the surface modification of prostheses and implants.

### Background art

In the field of prosthodontics and orthopedic joint prosthetics it has been found that the compatibility of the materials used for prostheses with biological tissues is fundamentally important to allow adequate treatment of the patients.

The materials commonly used in these fields consist substantially of metallic substrates of steel, titanium or tantalum, which have excellent mechanical properties but require long times for integration in the biological tissues of the patients in which they are implanted and for healing of the implantation region.

In order to obviate these drawbacks, methods have been developed which are suitable to modify the surface of the metallic substrates by introducing thereon chemical groups or chemical elements capable of interacting with biological tissues and thus allowing integration between the prosthesis and the tissues. These methods are generally known as biomimetic treatments.

For example, US patent 5,385,662 in the name of Kurze et al. describes the modification of metallic surfaces by means of a hydrogen peroxide treatment in order to deposit a layer of metallic oxides on said surfaces. This method, however, is not described for use in the biomedical field.

US patent 5,152,993 in the name of Lars-Magnus et al. and US patent 5,885,612 in the name of Ohthuki et al. describe the treatment of metallic surfaces by using hydrogen peroxide and optionally metallic ions, in order to modify the surfaces of metallic prostheses by introducing hydroxyl (-OH) groups thereon.

Patent EP0678300 in the name of Kokubo, on the other hand, describes the treatment of metallic surfaces by immersion in an alkaline solution of NaOH, followed by washing and high-temperature thermal treatment. This method, too, introduces -OH groups, which are suitable for interaction with bone tissues, on the treated metallic surface.

Moreover, US patent 5,478,237 in the name of Ishizawa et al. describes the use of anodic deposition on the surface of bone implants, with the object of modifying the composition and morphology of the metallic substrate. Although the introduction of -OH groups is not described, the process of US 5,478,237 leads to the forming of a porous layer that is rich in calcium and phosphorus on the surface of the implants, which facilitate their osteointegration.

Finally, patent EP1515759 in the name of Politecnico di Milano teaches the use of a double step of anodic deposition to deposit on the surface of metallic implants a microporous layer of calcium and phosphorus, at the same time introducing -OH groups.

Despite these technologies, there is nonetheless the need to provide new methods capable of facilitating integration of metallic substrates in biological tissues which are more efficient and simpler to perform.

### Summary of the invention

The main aim of the present invention is to provide metallic substrates modified with a biomimetic treatment.

Within the scope of this aim, an object of the invention is a surface modified metallic substrate with functional chemical groups and specific chemical elements, which can facilitate integration in biological tissues.

Another object of the invention is the use of said substrate for preparing prosthodontics and orthopedic joints prosthetics.

This aim and these and other objects, that will become better apparent hereinafter, are achieved by the surface modified metal substrates and their use as claimed in the appended claims. The surface modified metal substrates are selected from those belonging to the group consisting of titanium, tantalum, titanium alloys and tantalum alloys and are prepared with the method as disclosed and claimed in the parent application with publication Number EP2307594 (European regional phase of W02010013120) in particular comprising the steps:
(a) subjecting the metal substrate to an anodic spark deposition (ASD) treatment in an aqueous solution comprising sodium silicate hydrate (Na₂SiO₃·2H₂O), β-glycerophosphate (β-GP), calcium acetate hydrate (C₄H₆CaO₄·H₂O) and sodium hydroxide (NaOH); and
(b) immersing the metal substrate subjected to the ASD anodic deposition treatment in a solution of sodium hydroxide (NaOH).

### Brief description of drawings

Further characteristics and advantages of the invention will become better apparent from the detailed description of the method according to the invention and from the accompanying drawings, wherein:
Figure 1A is an SEM micrograph of a sample of titanium of the invention subjected to ASD treatment according to the method claimed in the aforementioned parent application, prior to immersion in NaOH;
Figure 1B is the spectrum obtained following EDS microanalysis of a sample of titanium of the invention prepared with ASD treatment according to the method claimed in the aforementioned parent application, prior to immersion in NaOH;
Figure 2A is an SEM micrograph of a sample of titanium of the invention prepared according to the method disclosed in the aforementioned and claimed in the aforementioned parent application.
Figure 2B is the spectrum obtained following EDS microanalysis of a sample of titanium of the invention prepared according to the method claimed in the aforementioned parent application.
Figure 3A is a chart of the amount of calcium (µg of Ca/cm² of sample) that is present on the samples subjected to ASD treatment according to the method disclosed and claimed in the aforementioned parent application, before (SUMNa no NaOH) and after (SUMNa) immersion in NaOH and the reference sample (BS);
Figure 3B is a chart of the amount of phosphorus (µg of P/cm² of sample) that is present on the samples subjected to ASD treatment according to the method claimed in the parent application, before (SUMNa no NaOH) and after (SUMNa) immersion in NaOH and on the reference sample (BS);
Figure 4A is an XRD diffractogram of a sample of the invention prepared according to the method claimed in the parent application (SUMNa);
Figure 4B is an XRD diffractogram of a reference sample (BS);
Figure 5A is a chart of the results of the profile analysis for the Rₐ parameter (data expressed as the mean ± standard deviation) of the sample of the invention prepared according to the method claimed in the aforementioned patent application (SUMNa) and the reference samples (Ti and BS);
Figure 5B is a chart of the results of the profile analysis for the Rₜ parameter (data expressed as the mean ± standard deviation) of a sample of the invention prepared according to the method claimed in the parent application (SUMNa) and the reference samples (Ti and BS);
Figure 5C is a chart of the results of the profile analysis for the Rₘₐₓ parameter (data expressed as the mean ± standard deviation) of a sample of the invention prepared according to the method claimed in the parent application (SUMNa) and the reference samples (Ti and BS);
Figure 5D is a table that summarizes the average values of the parameters Rₐ, Rₜ and Rₘₐₓ obtained from the profile analysis of the samples;
Figure 6 is a chart of the results of the Vickers microhardness test for samples prepared with the method claimed in the parent application following to ASD treatment according before (SUMNa wo NaOH) and after (SUMNa) immersion in NaOH and for the reference samples (Ti and BS);
Figure 7A is an SEM micrograph of a titanium sample according to the invention prepared with the method claimed in the parent application after the flexing test at 15° to the anchoring plane;
Figure 7B is an SEM micrograph of a titanium sample of the invention prepared according to the method claimed in the parent application after the flexing test at 45° to the anchoring plane;
Figure 8A is an SEM micrograph of a titanium reference sample (Ti) after 14 days of immersion in SBF;
Figure 8B is a spectrum obtained after EDS microanalysis of a titanium reference sample (Ti) after 14 days of immersion in SBF;
Figure 8C is an SEM micrograph of a titanium reference sample (BS) after 14 days of immersion in SBF;
Figure 8D is a spectrum obtained after EDS microanalysis of a titanium reference sample (BS) after 14 days of immersion in SBF;
Figure 8E is an SEM micrograph of a titanium sample of the invention prepared according to the method claimed in the parent application after 14 days of immersion in SBF;
Figure 8F is a spectrum obtained after EDS microanalysis of a titanium sample of the invention prepared with the method of the parent application after 14 days of immersion in SBF;
Figure 9A is an XRD diffractogram of a reference sample (Ti) after 14 days of immersion in SBF;
Figure 9B is an XRD diffractogram of a reference sample (BS) after 14 days of immersion in SBF;
Figure 9C is an XRD diffractogram of a sample of the invention prepared according to the method disclosed in the parent application (SUMNa) after 14 days of immersion in SBF;
Figure 10A is an optical metallograph of a reference sample (Ti) after 14 days of immersion in SBF;
Figure 10B is an optical metallograph of a reference sample (BS) after 14 days of immersion in SBF;
Figure 10C is an optical metallograph of a sample of the invention prepared with the method claimed in the parent application (SUMNa) after 14 days of immersion in SBF;
Figure 11 shows the results of the Alamar Blue assay after 7 days of culture of MG63 cells on samples subjected to ASD treatment according to the method claimed in the parent application before (SUMNa wo NaOH) and after (SUMNa) immersion in NaOH and on the reference samples (Ti and BS); the data, indicated as arbitrary units (ABUs), are derived from the mean of three different cultures for each sample ± standard deviation;
Figure 12A is an SEM micrograph of a reference sample (Ti) after 7 days of culture of MG63 cells;
Figure 12B is an SEM micrograph of a reference sample (BS) after 7 days of culture of MG63 cells;
Figure 12C is an SEM micrograph of a sample of the invention prepared according to the method claimed in the parent application (SUMNa) after 7 days of culture of MG63 cells;
Figure 13 is a chart of the content of CICP (type I C-terminal collagen sequence) on the samples subjected to ASD treatment according to the method claimed in the parent application before (SUMNa wo NaOH) and after (SUMNa) immersion in NaOH and on the reference samples (Ti and BS) after 7 days of culture of MG63 cells; the data are indicated as mean values on four samples standard deviation;
Figure 14 is a chart of the deposition of osteocalcin after 7 days of culture of MG63 cells on the samples subjected to ASD treatment according to the method claimed in the parent application before (SUMNa wo NaOH) and after (SUMNa) immersion in NaOH and on the reference samples (Ti and BS); the data are indicated as mean values on four samples ± standard deviation;
Figure 15 is a chart of the relation between fibronectin and serum albumin absorption on the samples subjected to ASD treatment according to the method claimed in the parent application before (SUMNa wo NaOH) and after (SUMNa) immersion in NaOH and on the reference samples (Ti and BS); the data, indicated as arbitrary units (ABUs), are derived from the mean of four samples ± standard deviation.

### Ways of carrying out the invention

In order to provide the metallic substrates with which prostheses and implants are made with better properties of integration in biological tissues, these substrates are modified by means of biomimetic treatments, bringing to a morphological and chemical modification of the surface of the metallic substrates by means of a series of passages of the method described in the parent Application.

The first passage of this method (step (a)) provides for use of the anodic spark deposition (ASD) technique, a technique for anodic passivation of metals by virtue of which it is possible to deposit on the metallic surface a layer of metallic oxide. Together with the ASD technique, an aqueous solution of chemical agents is used with which the surface of the metal substrates is modified, the metal being selected from the group consisting of titanium (Ti), tantalum (Ta), titanium alloys and tantalum alloys. Preferably, the metal substrate can be titanium.

In particular, during step (a) a metal substrate is subjected to ASD treatment in a deposition aqueous solution which comprises sodium silicate hydrate (Na₂SiO₃·2H₂O), β-glycerophosphate (β-GP), calcium acetate hydrate (C₄H₆CaO₄·H₂O) and sodium hydroxide (NaOH).

In one embodiment of the process claimed in the parent application, the deposition aqueous solution can comprise sodium silicate hydrate at a concentration of 0.005 M to 0.1 M, preferably 0.03 M, β -glycerophosphate at a concentration of 0.03 M to 0.2 M, preferably 0.1 M, calcium acetate hydrate at a concentration of 0.05 M to 0.6 M, preferably 0.3 M, and NaOH at a concentration of 0.005 M to 0.4 M, preferably 0.036.

Preferably, the ASD treatment step is performed at a temperature comprised in an interval of 0±0.5 °C.

In another embodiment, the ASD treatment step is preferably performed by working at a first current density value of 5 to 50 mA/cm², preferably 10 mA/cm², with a potential that increases freely up to a value of 210 to 310 V, preferably 300 V, for a period of time needed to reach said potential value and a second current density value of 50% to 5%, preferably 20%, of said first current density value.

The second phase of the above method (step (b)) consists in immersing the metal substrate subjected to the ASD anodic deposition treatment in a solution of sodium hydroxide (NaOH). This immersion leads to an etching produced by the alkaline solution on the surface of the metal, by virtue of which it is possible to optimize the quantitative ratio between the chemical elements deposited during step (a) and introduce - OH groups on the surface of the metal substrate.

Preferably, the immersion of the substrate in the NaOH solution after ASD anodic deposition can be performed at a temperature of 60±2 °C for a period of time from 0.25 to 6 hours, more preferably 2 hours. Moreover, the NaOH solution used can have a concentration of 0.5 to 10 M, preferably 5 M, of NaOH.

In another embodiment, the method disclosed in the parent application can furthermore comprise a step of cleaning the metal substrate before the anodic deposition step ASD, immersing the substrate in an ultrasound tray containing acetone for a first period of time from 3 to 5 minutes, and distilled water for a second period of time from 3 to 5 minutes.

This cleaning step is useful to remove any impurities on the surface of the metal substrate before performing the ASD treatment.

Furthermore, in another embodiment, the method claimed in the parent application can further comprise, independently of each other, the steps of cleaning the metal substrate by immersion in distilled water after the anodic deposition treatment and/or after the immersion of the substrate in the NaOH solution.

The step of further cleaning in distilled water after the ASD treatment and/or after immersion in the NaOH solution is useful to remove from the surface of the substrate any contaminating residues.

By performing steps (a) and (b) of the method claimed in the parent application it is therefore possible to obtain, in just two steps, the deposition on the surface of the metal substrate of a layer of metal oxide enriched with calcium (Ca), phosphorus (P), silicon (Si) and sodium (Na) that has a microporous, resistant, adherent morphology and at the same time is characterized by the presence of -OH chemical groups, thus providing the substrate with considerable biomimetic properties.

Preferably, the deposition aqueous solution used in the method disclosed and claimed in the parent application can comprise Na₂SiO₃·2H₂O at a concentration from 0.005 M to 0.1 M, preferably 0.03 M, β-GP at a concentration from 0.03 M to 0.2 M, preferably 0.1 M, C₄H₆CaO₄·H₂O at a concentration from 0.05 M to 0.6 M, preferably 0.3M, and NaOH at a concentration from 0.005 M to 0.4 M, preferably 0.036 M.

Advantageously, this deposition solution makes it possible to introduce on the surface of metallic substrates subjected to ASD anodic treatment chemical elements that are suitable to increase the biomimetic properties of the surface modified metallic substrate of the invention without resorting to multiple anodic deposition cycles.

### Example

Metallic samples of titanium were subjected to the method disclosed in the parent application according to the following procedure:
1. surface cleaning of the CP grade 2 titanium samples, by using an ultrasound tray, for 3-5 minutes in acetone and 3-5 minutes in distilled water;
2. ASD treatment in aqueous solution ofNa₂SiO₃·2H₂O 0.03 M, β -GP 0.1 M, C₄H₆CaO₄·H₂O 0.3 M, and NaOH 0.036 M; the solution is kept at approximately 0° ± 0.5 °C, current density of 10 mA/cm², potential free to rise to 300 V; the ASD treatment is ended when the potential value reaches 300 V and the current density drops to 20% of the set value;
3. washing in distilled water;
4. etching treatment with a 5 M alkaline solution of NaOH at a temperature of 60 ± 2 °C for 2 hours,
5. washing in distilled water and drying.

The samples obtained by means of the procedure described here, referenced hereinafter as SUMNa samples, were then compared with two distinct series of comparison samples. The first set of comparison samples consists of samples of untreated titanium, simply cleaned by using an ultrasound tray, for 3-5 minutes in acetone and 3-5 minutes in distilled water, referenced hereinafter as Ti samples. The second set of comparison samples is obtained by means of a process in three passages, which consists of: ASD treatment in a 0.015 M solution of calcium-glycerolphosphate (C₃H₇CaO₆P) at a current density of 7 mA/cm² up to a maximum voltage of 350 V; second ASD treatment in a 0.1 M solution of Ca(OH)₂ at a current density of 7 mA/cm² and at a maximum voltage of 370 V; thermal/chemical post-treatment in 5 M KOH at 60 ± 2 °C for 24 hours. The comparison samples of the second set are referenced hereinafter as BS samples.

The samples of the invention and the comparison samples have been examined and characterized by means of the following experimental procedures:
- Scanning electron microscopy (SEM): analysis of surface morphology (ZEISS-EVO 50 EP and Cambridge - Stereoscan 360). For the observation of the cells grown on the samples, the surface of each sample was coated with gold (Edwards, Sputter Coater S150B) and observation was performed under high vacuum conditions (1x10⁻⁵ mm Hg).
- Energy-dispersive X-ray spectroscopy (EDS): microanalysis (Oxford, Inca Energy 200) was performed on 500x magnifications on a portion of sample of micrometric size.
- Laser profilometry: the samples were subjected to 3 measurements of surface roughness performed with a laser profilometer (UBM Microfocus, model 5600).
- Thin film X-ray diffractometry (TF-XRD): the structure of the films was analyzed by means of a diffractometer (Philips PW 3710) with copper anode, setting 40 mA of current and a voltage of 40 kV. The thin film configuration was used for the present investigations.
- Plasma emission optical spectroscopy (ICP-OES): a quantitative composition analysis was performed on each individual specimen after etching with 5 ml of 96% sulfuric acid at 60 °C for 24 hours. Plasma emission spectroscopic analysis was performed on the resulting sample after dilution to 50 ml.
- Vickers microhardness analysis: microhardness tests were conducted by means of a microdurometer (Leitz, Wetzlar, Germany) while applying a 50 g load to the surface.
- Analysis of oxide adherence: rectangular samples of anodized titanium (30 x 70 mm) were bent at 15° and 45°. An analysis with an electron microscope was performed on the bent surface to assess damage to the surface film.
- Optical metallography: metallographic analyses were conducted on the samples after 14 days of incubation in Kokubo-type SBF solution, to assess the thickness of the deposit of any calcium phosphates that might be present.
- In vitro assays (to assess the bioactivity of the treatment): the treated samples were immersed in a suitable SBF (Simulated Body Fluid) solution to assess the growth and coating of calcium phosphates present in the solution. The presence and extent of said coatings is considered a significant indicator of bioactivity of the treatment and of the potential as a biomimetic treatment for osteointegration. The SBF solution used has the following composition (devised by Kokubo) per liter of solution: 8 g of NaCl, 0.353 g of NaHCO₃, 0.224 g of KCl, 0.231 g ofK₂HPO₄·3H₂O, 0.311g of MgCl₂·6H₂O, 0.368 g of CaCl₂·2H₂O, 0.071 g of Na₂SO₄. At the end, 6.057 g of tris-(hydroxymethyl)-aminomethane ((HOCH₂)₃CNH₂)tris) were added, and finally the pH was adjusted to 7.40 by adding HCl. Each sample was placed in a well of a multiple-well plate and covered with 12 ml of SBF solution. The plates, sealed with Parafilm, were placed in a stove at the constant temperature of 36.5±1.5 °C for 1, 5, 7, 11 and 14 days. Every 24 hours, the solution was replaced with new solution, preheated to 37 °C. The surface morphology of the samples was analyzed by SEM in order to determine the extent of any calcium phosphate deposits.

In vitro cellular assays used MG63 cells (European Collection of Animal Cell Cultures, ECACC) cultured with Minimum Essential Eagle Medium (EMEM) (M2279, Sigma) containing 10% (w/w) of fetal bovine serum (FBS) (Sigma), 1% (w/w) of nonessential amino acids (NEAA) (Sigma), 2 mM of glutamine (Sigma), 100 units/ml of penicillin (Sigma) and 0.1 mg/ml of streptomycin (Sigma) in an incubator (ThermoForma, mod3111) at 37 °C, relative humidity of 95% and 5% w/w of CO₂.
- Sterilization: the samples were sterilized by ultraviolet exposure after passing through 70% ethanol.
- Cell seeding: 50 µ1 (1x10⁴ cells) of the cellular suspension previously obtained were dispensed on each sample with a micropipette.
- Extraction of supernatants and cell lysates: for each time interval, the supernatant was taken from each sample and then cryopreserved at -80 °C. After washing in PBS, the intracellular and extracellular proteins were extracted from the samples. Centrifugation at 2000 rpm for 5 minutes yielded the cell lysate, which was subsequently cryopreserved at -80 °C.
- Direct cytotoxicity analysis (Alamar assay): under sterile conditions, for each time interval 1, 3 and 7 days, the culture medium was replaced with 500 µl of Alamar solution (10% Alamar Blue (w/w) in EMEM), which was left to incubate for 4 hours. 100 µl of supernatant containing Alamar Blue were thus dispensed in a multiple-well plate and a spectrophotometer (Tecan, model Genios Plus) was used to read absorbance at a wavelength of 570 nm with reference wavelength at 630 nm.
- Determination of total protein content: starting from the resulting cell lysates, the total content of protein present on each sample was quantified by means of a BCA™ Protein Assay Kit (Pierce).

Collagen I: CICP (C-Terminal Collagen I) EIA Kit ELISA (Metra-Quidel) was used to determine in the supernatant the level of C-terminal peptide released in stoichiometric quantity with respect to the production of Collagen I by the cells in the culture medium (supernatant) after 1, 3 and 7 days of culture.
- Osteocalcin: the level of osteocalcin deposited by the cells after 1, 3 and 7 days of culture was determined by means of a Gla-type Osteocalcin EIA Kit ELISA (Zymed) in the respective cell lysates.
- Protein adsorption in human serum: the samples were incubated at 37 °C with 300 µl of serum for 2 hours. After washing in PBS, the proteins were extracted by using an extraction buffer and a scalpel. Centrifugation at 2000 rpm for 5 minutes yielded the protein extract, subsequently cryopreserved at -80 °C.
- Determination of the total content of serum proteins: starting from the protein extracts, the content of serum proteins adsorbed on each sample was quantified by BCA™ Protein Assay Kit (Pierce).
- Fibronectin: the level of fibronectin adsorbed on the surfaces, after incubation in human serum, was investigated by means of a QuantiMatrix™ HUMAN FIBRONECTIN ELISA KIT (Chemicon) in the various protein extracts.
- Albumin: the level of albumin adsorbed on the surfaces, after incubation in human serum, was investigated by Western Blot. The proteins were separated by electrophoresis in 10% AcrylamideBisacrylamide gel (37.5/1) and then transferred onto a PVDF membrane. Incubation was performed with the primary antibody (Goat Polyclonal to Human Serum Albumin - ab 19180; Abcam) and finally with the secondary one (Peroxidase-conjugated AffinPure Mouse Anti Goat). The presence of albumin was detected by means of a Kodak Image Station.

The results of the analysis procedures are as follows.

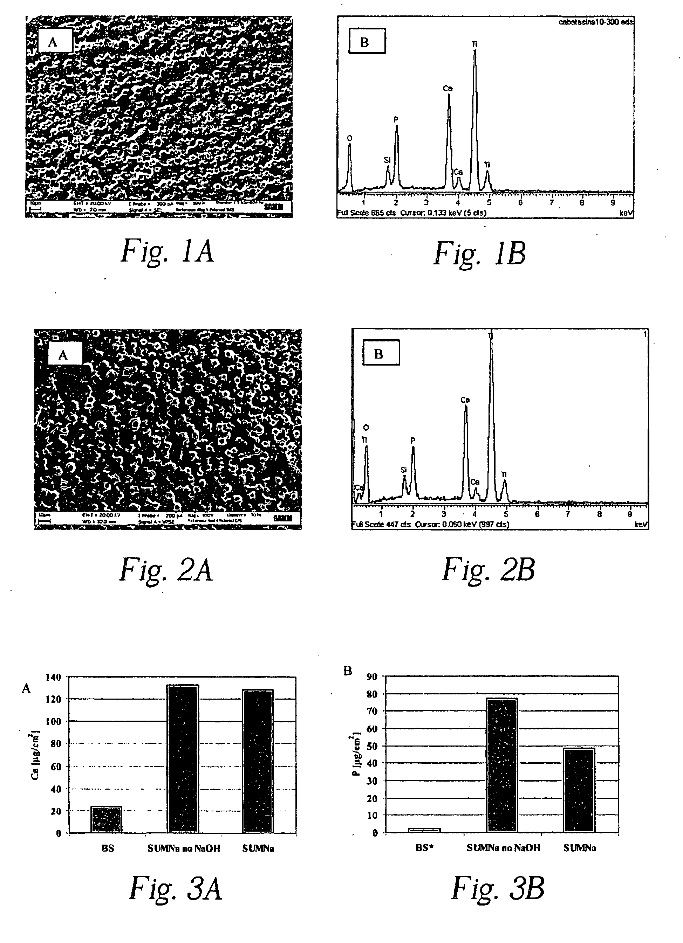

Analysis of surface morphology by SEM showed, for the surface of the SUMNa samples, both before (Figure 1A) and after (Figure 2A) immersion in NaOH, the presence of rounded micrometric pores; the appearance of the entire surface is uniform. Before immersion in NaOH (etching), EDS analysis highlights the presence on the surface of calcium, silicon and phosphorus (Figure 1B), whereas immersion in NaOH makes it possible to increase the calcium/phosphorus ratio and enrich the surface with sodium (Figure 2B).

ICP/OES analysis confirms what was found by EDS microanalyses. Figure 3A in fact makes it possible to see that the calcium and phosphorus quantities that are present on the SUMNa samples are higher than those present on the control BS. One can also notice that the etching in NaOH increases the calcium/phosphorus ratio.

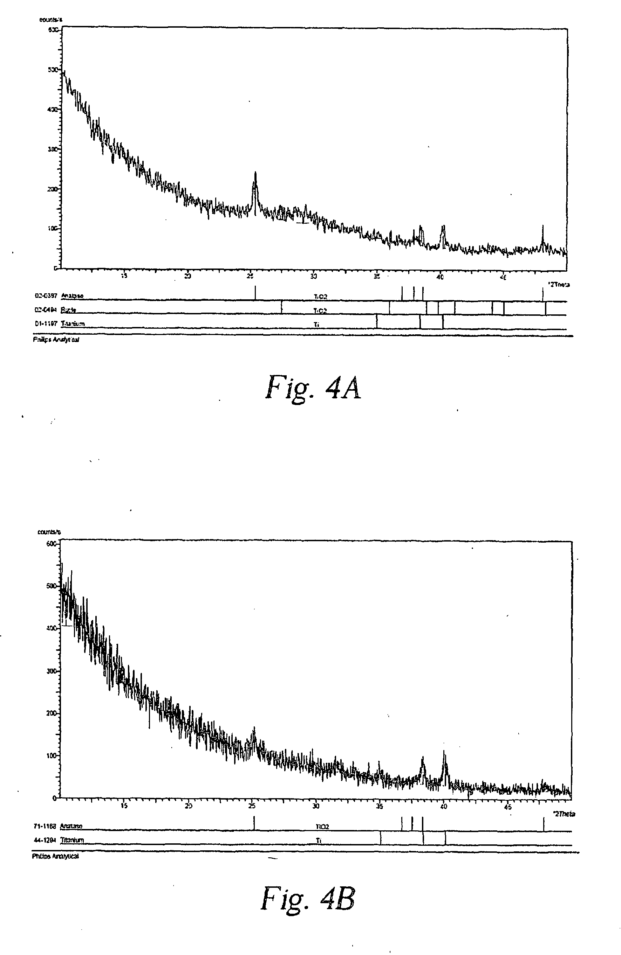

XRD analysis highlights that the titanium oxide that is present on the surface after the SUMNa treatment has the crystallographic structure of anatase, at a 20 angle of 25° (Figure 4A). This characteristic can be observed also on the control BS, although it is less conspicuous in the latter (Figure 4B). For the surface SUMNa, it can be noted that the anatase peak is higher than the titanium peak and therefore on the surface there is a thick oxide layer. This is a positive factor, since anatase has important catalytic properties, which in vitro stimulate nucleation of hydroxyapatite crystal, allowing a high performance in vivo. Moreover, it induces an important decrease in bacterial adhesion without compromising eukaryotic cell activity.

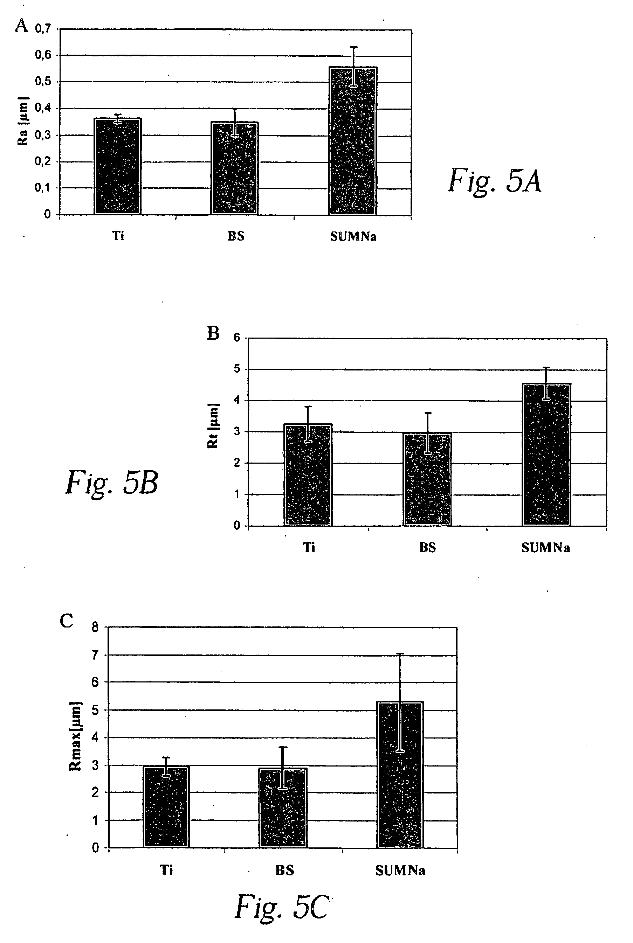

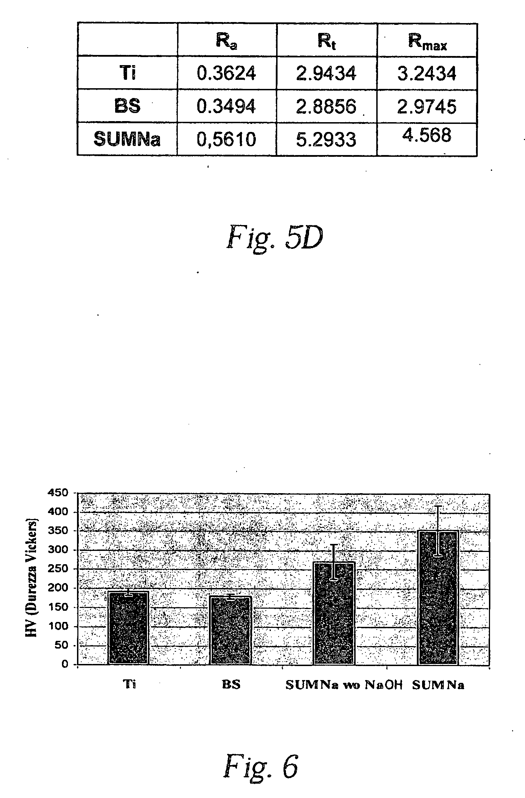

Laser profilometry showed, for the SUMNa samples, an increase in roughness (Rₐ, Rₘₐₓ and Rₜ) with respect to control materials (Figures 5A, 5B and 5C). It has been demonstrated extensively in literature that the surface roughness of a material affects cell behavior in terms of differentiation and proliferation. Profilometric analysis shows that SUMNa samples have a nano-rough surface.

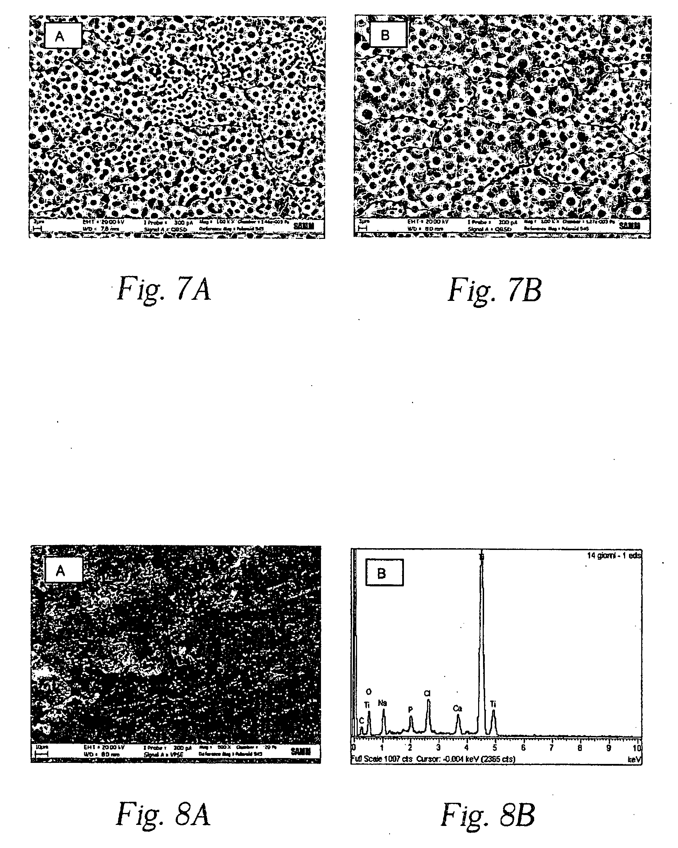

Vickers microhardness analysis shows that the surface of the samples prepared according to the method of the invention has a greater hardness than the control surfaces (Figure 6). This finding suggested an assessment whether the produced coating was fragile and susceptible of delamination, and therefore coating adherence tests were conducted with 15° and 45° flexing with respect to the anchoring plane of the specimen. Figure 7A shows that the surface exhibits slight cracks, which become more conspicuous after 45° flexing (Figure 7B). Despite the extreme flexing condition applied, the coating remains adherent to the surface of the underlying titanium, without exhibiting any delaminated region without oxide.

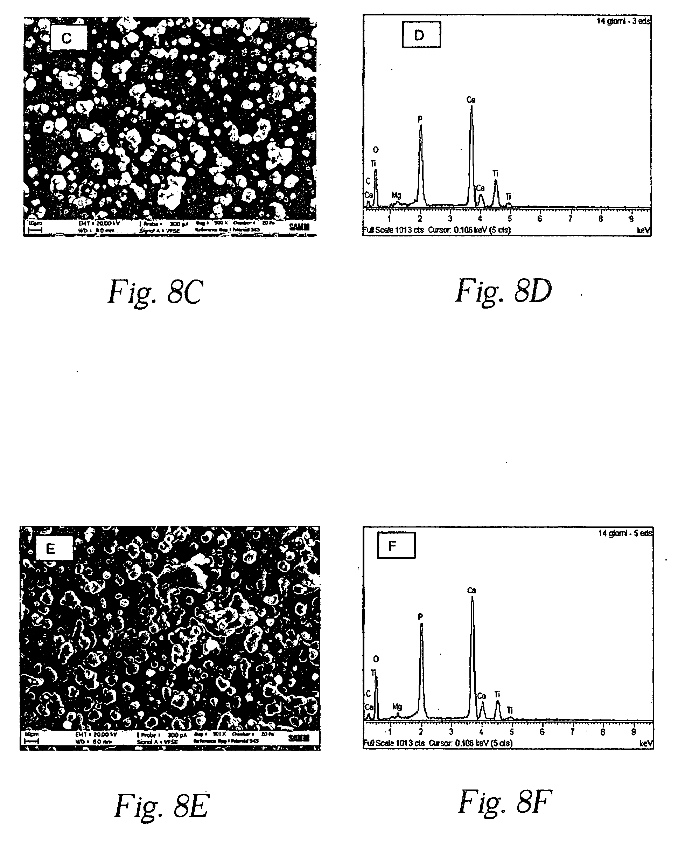

In order to establish the bioactivity of the coating obtained by means of the method according to the invention and to confirm the osteointegration capacities thereof, the quantity of calcium phosphates deposited on the SUMNa samples after 14 days of immersion in SBF solution was assessed. After 14 days of immersion, it is noted that the surfaces of the SUMNa samples show a higher quantity of calcium phosphates than the controls (Figures 8A, 8C and 8E). Furthermore, EDS analyses performed on the samples after 14 days of immersion in SBF show the presence of calcium and phosphorus on all the surfaces (Figures 8B, 8D and 8F). In particular, the untreated titanium sample (sample Ti) shows a far more intense titanium peak. This indicates that the calcium phosphate coating on the surface is limited (Figure 8B).

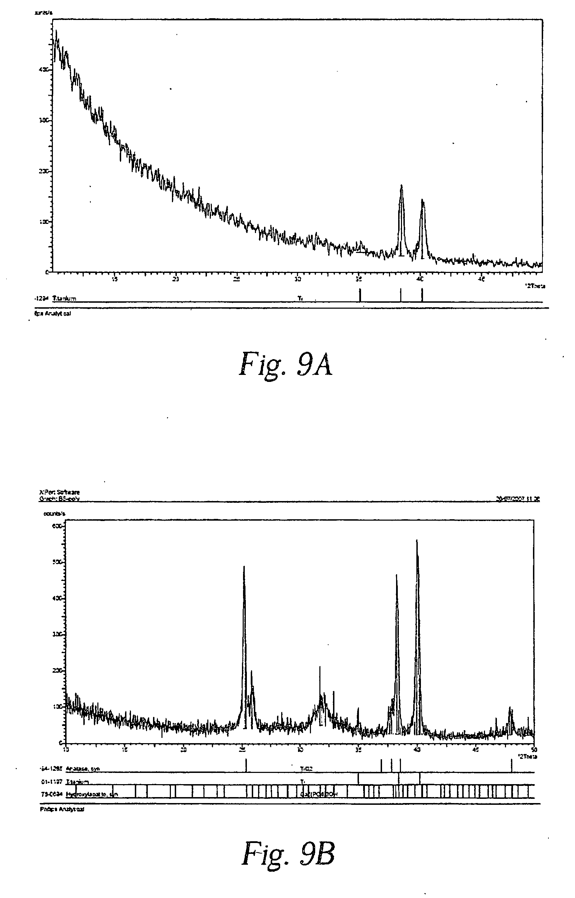

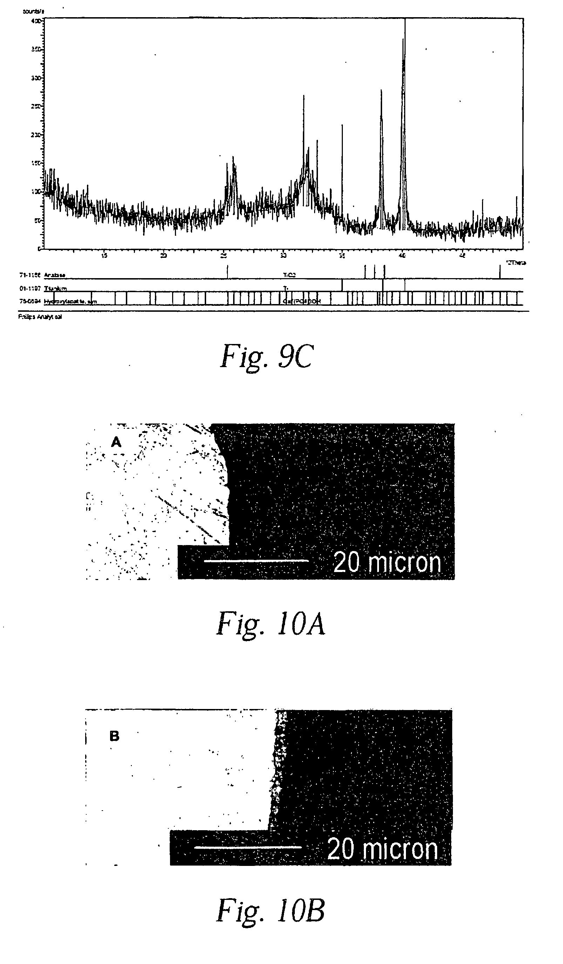

XRD analysis performed after 14 days of immersion of the samples in SBF shows the presence of hydroxyapatite (HA) on the surface of the SUMNa samples and of the BS samples, but not on the untreated titanium (Figures 9A, 9B and 9C). The peaks of the crystalline phase of HA are not distinctly highlighted due to the presence of HA in the amorphous phase.

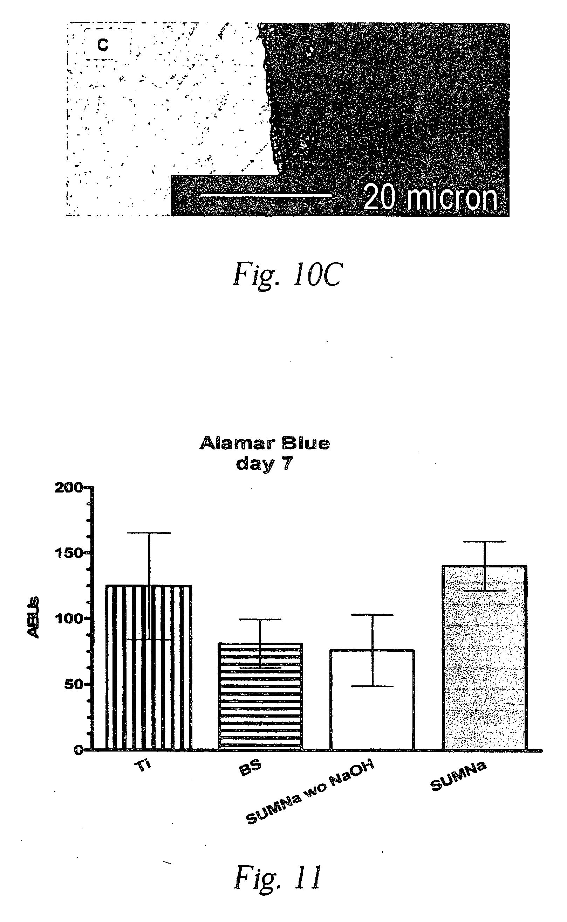

Metallographic investigations made it possible to improve the understanding of the extent of the mineralization phenomenon on each sample. These investigations do not show the presence of HA on the untreated titanium (Ti), reveal the presence of a layer of HA with a thickness of 5 µm on the BS samples and the presence of a far more bioactive surface on the SUMNa samples, with an HA thickness of 12 µm (Figures 10A, 10B and 10C). Therefore, the samples treated with the method according to the invention have a higher mineralization potential than the control BS and have chemical and morphological characteristics capable of inducing a higher deposition of calcium phosphates. This mechanism constitutes a fundamental step in the osteointegration phenomenon.

The cellular results obtained confirm that the surface subjected to SUMNa treatment is not cytotoxic, provides the cells with preferential anchor points for adhesion by virtue of the roughness of the surface, and constitutes a favorable environment for the occurrence of their metabolic activities.

The Alamar Blue assay shows that a higher cell vitality is observed with SUMNa samples than with the control BS. The treatment in NaOH undergone by the SUMNa samples is decisive in the increase in cell vitality. Prior to this alkaline etching, cell vitality on the SUMNa samples is in fact comparable to that of the controls (Figure 11). Treatment with alkaline solution in general intervenes by modifying both the morphological properties and the chemical properties of the coating: hydroxylation of the surface with enrichment of -OH groups is one of the typical effects produced.

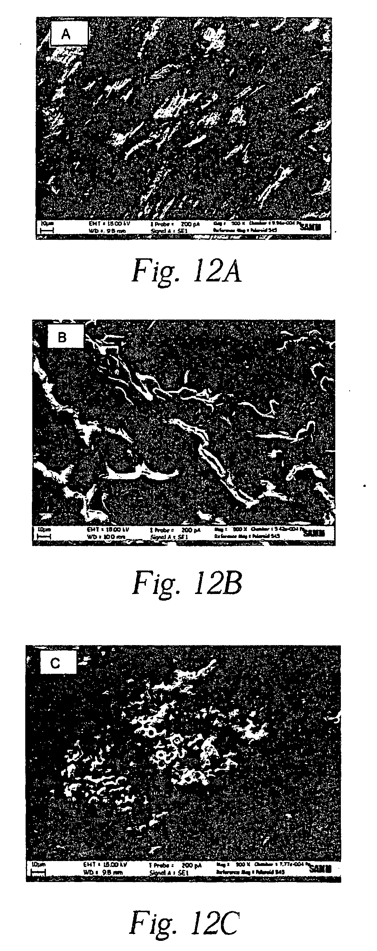

SEM analysis of cell morphology shows that MG63 cells have strongly adhered to the surface of the various materials. In particular, on the SUMNa and BS samples the cells form a uniform and multilayered structure (Figures 12B and 12C). Only isolated cell bodies are visible on the untreated titanium (Figure 12A). In view of the particular porous morphology of the SUMNa samples, it is difficult to be able to identify the cell bodies, since they blend in with the porosities (Figure 12C).

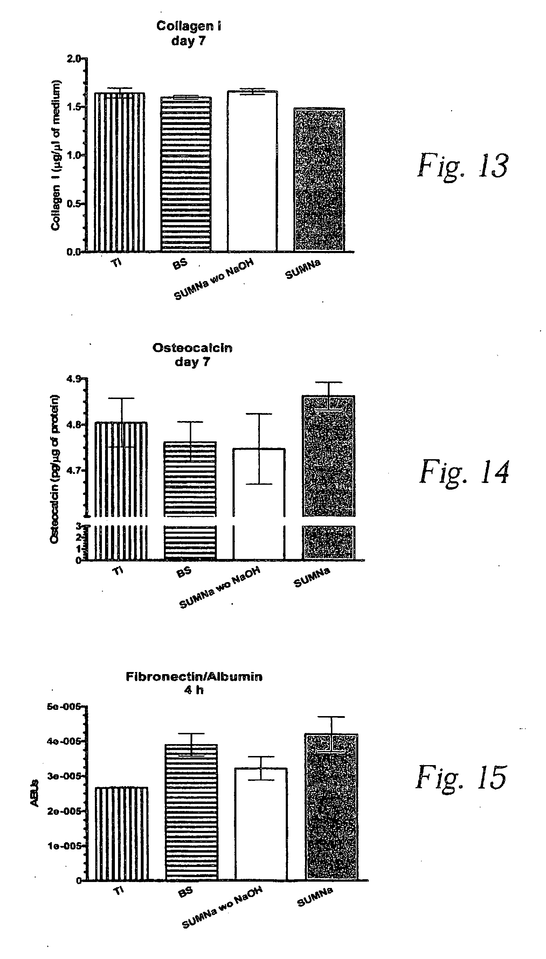

As regards the quantity of collagen I deposited by the cells after 7 days of cell culture, no significant variations among the various surfaces are observed (Figure 13).

The level of osteocalcin deposited by the MG63 cells after 7 days of culture on the various samples is higher on SUMNa samples than on the controls, which show a lower deposition (Figure 14). Since osteocalcin is one of the most significant biochemical markers of bone neoformation and remodeling, these data indicate that the method according to the present invention is the one that best promotes the mechanisms tied to cell differentiation and to osteogenesis.

Tests for protein adsorption in human serum were performed based on the fact that the first mechanism after implantation in a biological environment consists in contact between blood proteins and the surface of the material of the implant. The protein film that is deposited as a consequence of this first contact is fundamental in guiding the subsequent osteointegration process of the implant and cell response. The tests conducted investigated the levels of fibronectin and albumin adsorbed on the surfaces. The most interesting indicator is the ratio between fibronectin and albumin adsorbed; this indicator shows that the SUMNa samples have a selective and preferential adsorption of fibronectin with respect to the controls, thus entailing a higher osteointegration potential of the innovative treatment (Figure 15).

In practice it has been found possible to have surface modified metallic substrates to be used as prostheses, characterized by high capacities of integration in biological tissues.

Moreover, it has been found that the surface modified metallic substrate of the invention have both functional chemical groups and specific chemical elements capable of facilitating integration of the substrate in biological tissues and at the same time show a modified morphology, giving them a microporous structure.

Finally, it has been found that the substrates can be used as prostheses, since they are characterized by simplicity in execution and competitive costs.

## Claims

1. A substrate of a metal selected from the group consisting of titanium tantalum, titanium alloys and tantalum alloys, modified by deposition on the surface thereof of a layer of the oxide of the same metal, **characterized in that** said layer:
• is enriched with Ca, P , Si and Na,
• has a microporous resistant and adherent morphology,
• is provided with -OH chemical groups.

2. The substrate as claimed in claim 1, wherein said metal is titanium.

3. Use of the substrate as claimed in any of claims 1 - 2 for preparing prosthodontics and orthopedic joints prosthetics.

4. A prosthesis made of the surface modified metal substrate as claimed in anyone of claims 1- 2.

5. An implant made of the surface modified metal substrate as claimed in any one of claims 1 -2.

## Patentansprüche

1. Substrat aus einem Metall, ausgewählt aus der aus Titan, Tantal, Titanlegierungen und Tantallegierungen bestehenden Gruppe, das durch Abscheidung einer Schicht des Oxids desselben Metalls auf der Oberfläche davon modifiziert ist, **dadurch gekennzeichnet, daß** die Schicht:
- mit Ca, P, Si und Na angereichert ist,
- eine mikroporöse, resistente und haftende Morphologie aufweist,
- mit -OH-Gruppen versehen ist.

2. Substrat nach Anspruch 1, wobei das Metall Titan ist.

3. Verwendung des Substrates nach einem der Ansprüche 1 bis 2 zum Herstellen von Zahnprothesen und orthopädischen Gelenkprothesen.

4. Prothese, die aus dem oberflächenmodifizierten Metallsubstrat nach einem der Ansprüche 1 bis 2 hergestellt ist.

5. Implantat, das aus dem oberflächenmodifizierten Metallsubstrat nach einem der Ansprüche 1 bis 2 hergestellt ist.

## Revendications

1. Substrat en un métal choisi dans le groupe constitué par le titane, le tantale, les alliages de titane et les alliages de tantale, modifié par dépôt, sur la surface de celui-ci, d'une couche de l'oxyde du même métal, **caractérisé par le fait que** ladite couche :
- est enrichie en Ca, P, Si et Na,
- présente une morphologie microporeuse résistante et adhérente,
- est pourvue de groupes -OH.

2. Substrat selon la revendication 1, dans lequel ledit métal est le titane.

3. Utilisation du substrat selon l'une quelconque des revendications 1 à 2, pour la fabrication de prothèses dentaires et de prothèses articulaires orthopédiques.

4. Prothèse réalisée à partir du substrat métallique à surface modifiée selon l'une quelconque des revendications 1 à 2.

5. Implant réalisé à partir du substrat métallique à surface modifiée selon l'une quelconque des revendications 1 à 2.
